# EUROPÄISCHE PATENTSCHRIFT

(11) **EP 1 289 559 B1**
(45) Veröffentlichungstag und Bekanntmachung des Hinweises auf die Patenterteilung: **27.07.2005**
(21) Anmeldenummer: 01945184.8
(22) Anmeldetag: 22.05.2001
(51) Int. Cl.: A61K 45/06, A61P 37/06, A61P 35/00

(54) **KOMBINATIONEN VON ENZYMINHIBITOREN UMFASSENDE ZUBEREITUNGEN UND DEREN VERWENDUNG**
COMBINATIONS OF ENZYME INHIBITOR-CONTAINING PREPARATIONS AND THE USE THEREOF
PREPARATIONS CONTENANT DES COMBINAISONS D'INHIBITEURS ENZYMATIQUES, ET LEURS UTILISATIONS

(30) Priorität: 23.05.2000 DE 10025464
(43) Veröffentlichungstag der Anmeldung: 12.03.2003
(73) Patentinhaber: Institut für Medizintechnologie Magdeburg GmbH IMTM., 39120 Magdeburg (DE)
(72) Erfinder: ANSORGE, Siegfried, 39291 Hohenwarthe (DE); ARNDT, Marco, 06217 Merseburg (DE); BÜHLING, Frank, 39108 Magdeburg (DE); LENDECKEL, Uwe, 39128 Magdeburg (DE); NEUBERT, Klaus, 06128 Halle/Saale (DE); REINHOLD, Dirk, 39108 Magdeburg (DE); BROCKE, Stefan, 12053 Berlin (DE)
(74) Vertreter: Koepe, Gerd L., Dipl.-Chem.
(86) Internationale Anmeldenummer: PCT/EP2001/005887
(87) Internationale Veröffentlichungsnummer: WO 2001/089569

(56) Entgegenhaltungen:
- STEINMETZER T ET AL: "Peptidyl ammonium methyl ketones as substrate analog inhibitors of proline-specific peptidases." JOURNAL OF ENZYME INHIBITION, (1993) 7 (2) 77-85. , XP001018795
- ANSORGE S ET AL: "Membrane-bound peptidases of lymphocytes: functional implications." BIOMEDICA BIOCHIMICA ACTA, (1991) 50 (4-6) 799-807. , XP001021975
- SHIMAZAWA R ET AL: "Novel small molecule nonpeptide aminopeptidase n inhibitors with a cyclic imide skeleton." JOURNAL OF ENZYME INHIBITION, (1999) 14 (4) 259-75. REF: 67 , XP001018772
- AUGUSTYNS K ET AL: "THE UNIQUE PROPERTIES OF DIPEPTIDYL-PEPTIDASE IV (DPP IV/CD26) AND THE THERAPEUTIC POTENTIAL OF DPP IV INHIBITORS" CURRENT MEDICINAL CHEMISTRY, BENTHAM SCIENCE PUBLISHERS BV, BE, Bd. 6, Nr. 4, 1999, Seiten 311-327, XP000870290 ISSN: 0929-8673
- REINHOLD D ET AL: "INHIBITORS OF DIPEPTIDYL PEPTIDASE IV INDUCE SECRETION OF TRANSFORMING GROWTH FACTOR-BETA1 IN PWM-STIMULATED PBMC AND T CELLS" IMMUNOLOGY, BLACKWELL SCIENTIFIC PUBLICATIONS, GB, Bd. 91, Nr. 3, 1997, Seiten 354-360, XP000867395 ISSN: 0019-2805 in der Anmeldung erwähnt

## Beschreibung

Die Erfindung beschreibt die kombinierte Hemmung von Enzymaktivitäten der Aminopeptidase N (APN, EC3.4.11.2, CD13), der Dipeptidylpeptidase IV (DP IV, EC 3.4.14.5, CD26), der Prolyloligopeptidase (POP, Prolylendopeptidase, PEP, EC3.4.21.26), der membranständigen Aminopeptidase P (X-Pro-Aminopeptidase, APP, XPNPEP2, EC 3.4.11.9) und des Angiotensin-konvertierenden Enzyms, (angiotensin-converting enzyme, ACE, EC 3.4.15.1, CD156) durch die simultane Applikation von jeweils spezifischen Inhibitoren dieser Enzyme auf der Basis von Aminosäurederivaten, Peptiden oder Peptidderivaten, durch welche die Aktivierung, die DNS-Synthese und damit die Proliferation von Immunzellen supprimiert wird.

Für alle Erkrankungen mit Autoimmunpathogenese gilt, dass eine Aktivierung und Proliferation von Immunzellen, insbesondere von autoreaktiven T-Zellen, dem Krankheitsprozess zugrunde liegen bzw. diesen ausmachen. Die gleichen Mechanismen kommen bei akuten bzw. chronischen Abstoßungsepisoden nach Organtransplantation zur Wirkung.

Es ist gezeigt worden, dass im Prozess der Aktivierung und klonalen Expansion von Immunzellen, insbesondere von T-Lymphozyten, membranständige Peptidasen wie DP IV oder APN eine Schlüsselrolle spielen [Fleischer B: CD26 a surface protease involved in T-cell activation. Immunology Today 1994; 15:180-184; Lendeckel U et al.: Role of alanyl aminopeptidase in growth and function of human T cells. International Journal of Molecular Medicine 1999; 4:17-27; Riemann D et al.: CD13 - not just a marker in leukemia typing. Immunology Today 1999; 20:83-88]. Verschiedene Funktionen mitogen-stimulierter mononukleärer Zellen (MNZ) oder angereicherter T-Lymphozyten wie DNS-Synthese, Produktion und Sekretion von immunstimulierenden Zytokinen (IL-2, IL-6, IL-12, IFN-γ) und Helferfunktionen für B-Zellen (IgG- und IgM-Synthese) können in Gegenwart von spezifischen Inhibitoren der DP IV und der APN gehemmt werden [Schön E et al.: The dipeptidyl peptidase IV, a membrane enzyme involved in the proliferation of T lymphocytes. Biomed. Biochim. Acta 1985; 2: K9-K15; Schön E et al.: The role of dipeptidyl peptidase IV in human T lymphocyte activation. Inhibitors and antibodies against dipeptidyl peptidase IV suppress lymphocyte proliferation and immunoglobulin synthesis in vitro. Eur. J. Immunol. 1987; 17: 1821-1826; Reinhold D et al.: Inhibitors of dipeptidyl peptidase IV induce secretion of transforming growth factor β1 in PWM-stimulated PBMNC and T cells. Immunology 1997; 91: 354-360; Lendeckel U et al. : Induction of the membrane alanyl aminopeptidase gene and surface expression in human T-cells by mitogenic activation. Biochem. J. 1996; 319: 817-823; Kähne T et al.: Dipeptidyl peptidase IV: A cell surface peptidase involved in regulating T cell growth (Review). Int. J. Mol. Med. 1999; 4: 3-15; Lendeckel U et al.: Role of alanyl aminopeptidase in growth and function of human T cells (Review). Int. J. Mol. Med. 1999; 4: 17-27].

Die Druckschrift "T. Steinmetzer et al.; J. Enzyme Inhibition 7 (1993), 77 - 85" betrifft die Inhibierung sowohl von DPIV als auch von POP durch dieselben Inhibitoren, nämlich Peptidyl-Ammonium-Methylketone.

Die Druckschrift "S. Ansorge et al.; Biomed. Biochim Acta 50 (191), 4 - 6" beschreibt die Rolle zweier Enzyme, nämlich der beiden Peptidasen DPIV und APN, im Organismus sowie ihre getrennte Inhibierung durch für diesen Zweck an sich bekannte Inhibitoren.

Die Druckschrift "R. Shimazawa et al.; J. Enzyme Inhibition 14 (1999), 259 - 275" beschreibt die Eigenschaft von Molekülen mit einem cyclischen Imid-Grundgerüst als Aminopeptidase-Inhibitoren. In diesem Dokument werden Moleküle (beispielsweise N-Phenylphthalimide) als Inhibitoren sowohl von DPIV als auch von APN beschrieben.

Es ist bereits bekannt, dass die Behandlung von Autoimmunerkrankungen und Abstoßungsreaktionen nach Transplantationen durch die Hemmung der auf Immunzellen lokalisierten Dipeptidylpeptidase IV mittels synthetischer Inhibitoren teilweise möglich ist (z.B. EP764151A1, WO09529691, EP731789A1, EP528858). Für die Enzyme Aminopeptidase N, "angiotensin-converting enzyme", X-Pro-Aminopeptidase und Prolyloligopeptidase sind solche Effekte bisher nicht bekannt

Der Erfindung liegt der überraschende Befund zugrunde, dass die gleichzeitige Hemmung der enzymatischen Aktivitäten von (I) Dipeptidylpeptidase IV und Aminopeptidase N, (II) der Dipeptidylpeptidase IV und des "angiotensin-converting enzym", (III) der Dipeptidylpeptidase IV und der Prolyloligopeptidase sowie (IV) der Dipeptidylpeptidase IV und der X-Pro-Aminopeptidase die DNS-Synthese und damit die Proliferation von mononukleären Zellen (MNZ) als auch von T-Zellen in einem Ausmaß hemmt, das durch die einzelne Applikation dieser Enzyminhibitoren - auch bei höherer Dosierung - nicht erreicht werden kann. Obgleich die genannten Inhibitoren letztendlich den gleichen Prozess, nämlich die DNS-Synthese und damit die Proliferation von Immunzellen, beeinflussen, ist dieser Effekt bei einzelner Applikation der Inhibitoren wesentlich schwächer ausgeprägt und nicht dauerhaft. Wegen der funktionellen Überschneidung der enzymatischen Aktivitäten der genannten Enzyme resultiert, wie unsere Daten zeigen, eine superadditive Hemmwirkung auf DNS-Synthese und Proliferation aus der gleichzeitigen Hemmung von zwei oder mehreren dieser Enzyme.

Unsere Erfindung zeigt, dass zur Therapie von Autoimmun- und entzündlichen Erkrankungen sowie zur Behandlung von Abstoßungsepisoden nach Transplantation die gleichzeitige Applikation von Hemmstoffen der oben genannten Enzyme bzw. entsprechender Zubereitungen und Darreichungsformen daraus geeignet sind.

Im einzelnen liegen der Erfindung die Befunde zugrunde, dass die DNS-Synthese von MNZ und T-Zellen durch die simultane Administration von Inhibitoren der enzymatischen Aktivität von
**I**. Dipeptidylpeptidase IV und Aminopeptidase N,
**II**. Dipeptidylpeptidase IV und Angiotensin-konvertierendem Enzym,
**III**. Dipeptidylpeptidase IV und Prolyloligopeptidase
**IV**. Dipeptidylpeptidase IV und X-Pro-Aminopeptidase
in superadditiver Weise inhibiert wird.

Die Applikation von Enzyminhibitoren stellt bei den genannten Erkrankungen eine neuartige Methode und ergänzende Therapie form dar.

Die Erfindung betrifft die Verwendung von Inhibitoren der Dipeptidylpeptidase IV (DP IV) sowie von Inhibitoren von Enzymen mit gleicher Substratspezifität (DP IV-analoge Enzymaktivität) in Kombination mit Inhibitoren der Alanyl-Aminopeptidase (Aminopeptidase N, APN) bzw. mit Inhibitoren von Enzymen gleicher Substratspezifität (APN-analoge Enzymaktivität), Inhibitoren der X-Pro-Aminopeptidase (Aminopeptidase P, APP), Inhibitoren des "angiotensin-converting enzyme" (ACE) und/oder Inhibitoren der Prolyloligopeptidase (POP, Prolylendopeptidase, PEP) zur Herstellung eines Arzneimittels zur Vorbeugung und Therapie von Autoimmunerkrankungen und/oder zur Unterdrückung von Transplantat-Abstossung und/oder zur Therapie von Tumorerkrankungen.

Die Erfindung betrifft auch pharmazeutische Zubereitungen, umfassend Inhibitoren der Dipeptidylpeptidase IV (DP IV) oder Inhibitoren von Enzymen mit DP IV-analoger Enzymaktivität in Kombination mit Inhibitoren eines der Enzyme Alanyl-Aminopeptidase (Aminopeptidase N, APN) bzw. Enzyme gleicher Substratspezifität (APN-analoge Enzymaktivität), X-Pro-Aminopeptidase (Aminopeptidase P, APP), "angiotensin-converting enzyme" (ACE) und/oder Prolyloligopeptidase (POP, Prolylendopeptidase, PEP) und in Kombination mit an sich bekannten Träger-, Zusatz- und/oder Hilfsstoffen.

Entsprechend der erfindungsgemäßen Verwendung sind die Inhibitoren der DP IV Xaa-Pro-Dipeptide (Xaa = α-Aminosäure bzw. seitenkettengeschütztes Derivat), entsprechende Derivate, vorzugsweise Dipeptidphosphonsäurediarylester, Dipeptidboronsäuren (z.B. Pro-boro-Pro) und deren Salze, Xaa-Xaa-(Trp)-Pro-(Xaa)n-Peptide (Xaa = α-Aminosäure, n=0-10), entsprechende Derivate und deren Salze bzw. Aminosäure (Xaa)-amide, entsprechende Derivate und deren Salze, wobei Xaa eine α-Aminosäure bzw. ein seitenkettengeschütztes Derivat, vorzugsweise N^{ε}-4-Nitrobenzyloxycarbonyl-L-Lysin, L-Prolin, L-Tryptophan, L-Isoleucin, L-Valin ist und als Amidstruktur cyclische Amine, z.B. Pyrrolidin, Piperidin, Thiazolidin und deren Derivate fungieren.

Gemäß einer bevorzugten Verwendung werden als Inhibitoren der DPIV Aminosäureamide, z.B. N^{ε}-4-Nitrobenzyloxy-carbonyl-L-Lysin-thiazolidid, -pyrrolidid und -piperidid sowie das entsprechende 2-Cyanothiazolidid-, 2-Cyanopyrrolidid- und 2-Cyanopiperidid-derivat eingesetzt.

Entsprechend der erfindungsgemäßen Verwendung werden als Inhibitoren der APN Actinonin, Leuhistin, Phebestin, Amastin, Probestin β-Aminothiole, α-Aminophosphinsäuren, α-Amino-phosphinsäurederivate, vorzugsweise D-Phe-ψ[PO(OH)-CH₂]-Phe-Phe und deren Salze, als Inhibitoren der APP Apstatin, (2S,3R)-HAMH-L-Prolin, (2S,3R)-HAPB-L-Prolin, die entsprechenden L-Prolinmethylester, (2S,3R)-HAMH/(2S,3R)-HAPB-pyrrolidide, -thiazolidide (HAMH = 3-Amino-2-hydroxy-5-methyl-hexanoyl, HAPB = 3-Amino-2-hydroxy-4-phenyl-butanoyl) und der Salze, als Inhibitoren des ACE Captopril, Enalapril, Lisinopril, Cilazopril und deren Salze, als Inhibitoren der POP (PEP) Postatin, Eurystatin A oder B, N^{a}-geschützte Peptidaldehyde, vorzugsweise Benzyloxycarbonyl-L-Prolyl-L-Prolinal bzw. Benzyloxycarbonyl-L-Thioprolyl-L-Thioprolinal, N^{a}-geschützte Aminosäure(Xaa)-pyrrolidide bzw. -thiazolidide (Xaa = α-Aminosäure, bevorzugt L-Alanin, L-Valin, L-Isoleucin) sowie die entsprechenden 2-Cyanopyrrolidid- bzw. 2-Cyanothiazolididderivate, substratanaloge N^{a}-geschützte Peptidphosphonsäurediarylester bzw. Peptiddiazomethylketone bzw. Peptidammoniummethylketone und deren Salze eingesetzt.

Entsprechend der erfindungsgemäßen Verwendung werden Inhibitorkombinationen gemäß der vorstehenden Angaben eingesetzt zur Vorbeugung und Therapie von Autoimmunerkrankungen und/oder zur Unterdrückung von Transplantat-Abstoßung und/oder zur Therapie von Tumorerkrankungen. Bevorzugt finden Sie Anwendung zur Vorbeugung und Therapie von Rheumatoider Arthritis, Lupus erythematodes, Multipler Sklerose, IDDM, Morbus Crohn, Colitis Ulcerosa, Psoriasis, Neurodermitis, Glumerulonephritis, interstitieller Nephritis, Vaskulitis, autoimmunen Schilddrüsenerkrankungen oder autoimmunhämolytischer Anämie, sowie anderen chronischen Erkrankungen mit entzündlicher Genese wie Allergien und Arteriosklerose.

Die pharmazeutischen Zubereitungen gemäß der Erfindung umfassen als Inhibitoren der DP IV vorzugsweise Xaa-Pro-Dipeptide (Xaa = α-Aminosäure bzw. seitenkettengeschützte Derivate), entsprechende Derivate, vorzugsweise Dipeptidphosphonsäurediarylester, Dipeptidboronsäuren (z.B. Pro-boro-Pro) und deren Salze, Xaa-Xaa-(Trp)-Pro-(Xaa)n-Peptide (Xaa = α-Aminosäuren, n=0-10), entsprechende Derivate und deren Salze bzw. Aminosäure (Xaa)-amide, entsprechende Derivate und deren Salze, wobei Xaa eine α-Aminosäure bzw. deren seitenkettengeschütztes Derivat, vorzugsweise N^{ε}-4-Nitrobenzyloxycarbonyl-L-Lysin, L-Prolin, L-Tryptophan, L-Isoleucin, L-Valin, ist und als Amidstruktur cyclische Amine, z.B. Pyrrolidin, Piperidin, Thiazolidin und deren Derivate fungieren. Derartige Verbindungen und deren Herstellung wurde in einem früheren Patent (DD 296 075 A5; Neubert et al.) beschrieben. Weiter bevorzugt umfassen die pharmazeutischen Zubereitungen als Inhibitoren der DPIV Aminosäureamide, z.B. N^{ε}-4-Nitrobenzyloxycarbonyl-L-Lysin-thiazolidid, -pyrrolidid und -piperidid sowie das entsprechende 2-Cyanothiazolidid-, 2-Cyano-pyrrolidid- und 2-Cyanopiperididderivat.

Die pharmazeutischen Zubereitung umfassen vorzugsweise die Inhibitoren Actinonin, Leuhistin, Phebestin, Amastin, Probestin, β-Aminothiole, α-Aminophosphinsäuren, α-Aminophosphinsäurederivate bevorzugt D-Phe-ψ[PO(OH)-CH₂]-Phe-Phe und deren Salze als APN-Inhibitoren, Apstatin, (2S,3R)-HANM-L-Prolin, (2S,3R)-HAPB-L-Prolin, die entsprechenden L-Prolinmethylester, (2S,3R)-HAMH-/(2S,3R)-HAPB-pyrrolidide, -thiazolidide (HAMH = 3-Amino-2-hydroxy-5-methyl-hexanoyl, HAPB = 3-Amino-2-hydroxy-4-phenyl-butanoyl) und der Salze als APP-Inhibitoren, Captopril, Enalapril, Lisinopril, Cilazopril und deren Salze als ACE-Inhibitoren, Postatin, Eurystatin A oder B, N^{a}-geschützte Peptidaldehyde, vorzugsweise Benzyloxycarbonyl-L-Prolyl-L-Prolinal bzw. Benzyl-oxycarbonyl-L-Thioprolyl-L-Thioprolinal, N^{a}-geschützte Aminosäure(Xaa)-pyrrolidide bzw. -thiazolidide (Xaa = α-Aminosäure, bevorzugt L-Alanin, L-Valin, L-Isoleucin) sowie die entsprechenden 2-Cyanopyrrolidid- bzw. 2-Cyanothiazolididderivate, substratanaloge N^{a}-geschützte Peptidphosphonsäurediarylester bzw. Peptiddiazomethylketone bzw. Peptidammoniummethylketone und deren Salze als POP (PEP)-Inhibitoren.

Weiter bevorzugt umfassen die erfindungsgemäßen pharmazeutischen Zubereitungen zwei oder mehrere der Inhibitoren der DP IV bzw. DP IV-analoger Enzymaktivität, der APN bzw. APN-analoger Enzymaktivität, des ACE, der POP (PEP) und der APP in räumlich getrennter Formulierung in Kombination mit an sich bekannten Träger-, Hilfs- und/oder Zusatzstoffen zur gleichzeitigen oder zeitlich unmittelbar aufeinanderfolgenden Verabreichung mit dem Ziel einer gemeinsamen Wirkung.

Die pharmazeutischen Zubereitungen gemäß der Erfindung sind weiter bevorzugt geeignet für die systemische Anwendung zur oralen, transdermalen, intravenösen, subcutanen, intracutanen, intramuskulären, rektalen, vaginalen, sublingualen Applikation zusammen mit an sich bekannten Träger-, Hilfs- und/oder Zusatzstoffen, oder sie sind geeignet für die topische Anwendung in Form von z.B. Cremes, Salben, Pasten, Gelen, Lösungen, Sprays, Liposomen, Schüttelmixturen, Hydrokolloidverbänden bzw. andem dermatologischen Grundlagen/Vehikeln, einschließlich instillativer Applikation.

Die erfindungsgemäß applizierten Inhibitoren der Dipeptidylpeptidase IV, der Aminopeptidase N, der Prolyloligopeptidase, des "angiotensin-converting enzym" und der X-Pro-Aminopepridase können in pharmazeutisch anwendbaren Formulierungskomplexen als Inhibitoren, Substrate, Pseudosubstrate, inhibitorisch wirkende Peptide und Peptidderivate sowie als Antikörper dieses Enzyms zur Anwendung kommen. Bevorzugte Effektoren sind beispielsweise für die DP IV Xaa-Pro-Dipeptide, entsprechende Derivate, vorzugsweise Dipeptidphosphonsäurediarylester und deren Salze, Xaa-Xaa-(Trp)-Pro-(Xaa)n-Peptide (n=0-10), entsprechende Derivate und deren Salze bzw. Aminosäure (Xaa)-amide, entsprechende Derivate und deren Salze, wobei Xaa eine α-Aminosäure/Iminosäure bzw. ein α-Aminosäurederivat/Iminosäurederivat, vorzugsweise N^{ε}-4-Nitrobenzyloxycarbonyl-L-Lysin, L-Prolin, L-Tryptophan, L-Isoleucin, L-Valin ist und als Amidstruktur cyclische Amine, z.B. Pyrrolidin, Piperidin, Thiazolidin und deren Derivate. Derartige Verbindungen und deren Herstellung wurden in einem früheren Patent beschrieben (K Neubert et al. DD296075A5).

Die Inhibitoren werden simultan mit bekannten Trägerstoffen verabreicht. Die Verabreichung erfolgt einerseits als topische Applikation in Form von z.B. Cremes, Salben, Pasten, Gelen, Lösungen, Sprays, Liposomen, Schüttelmixturen, Hydrokolloidverbänden bzw. anderen dermatologischen Grundlagen/Vehikeln einschließlich instilativer Applikation und andererseits als systemische Applikation zur oralen, transdermalen, intravenösen, subcutanen, intracutanen, intramuskulären Anwendung in geeigneten Rezepturen bzw. in geeigneter Galenik.

### Ausführungsbeispiele

### Beispiel 1

### Inhibierung der DNS-Synthese von humanen T-Lymphozyten durch Inkubation mit synthetischen Inhibitoren der DP IV und der APN

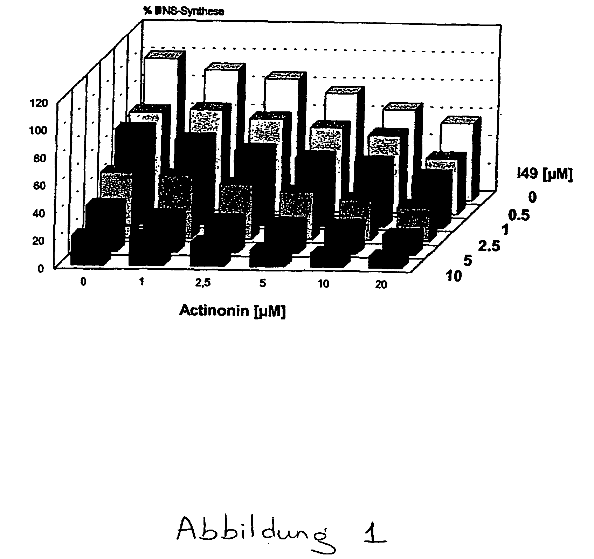

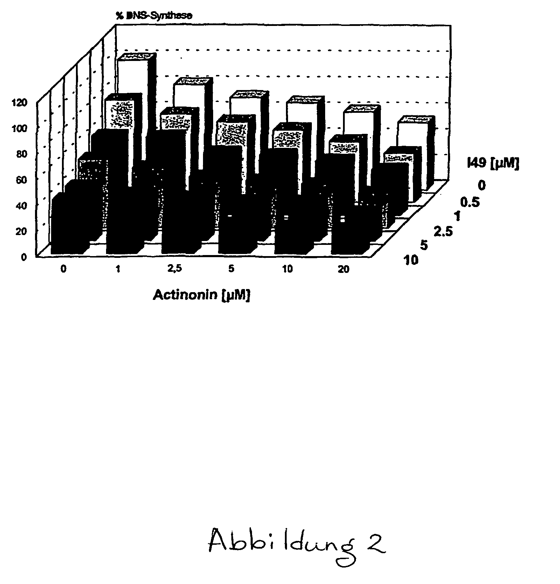

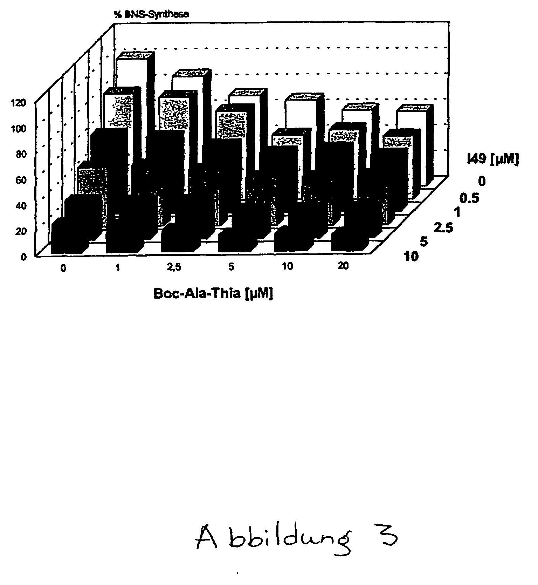

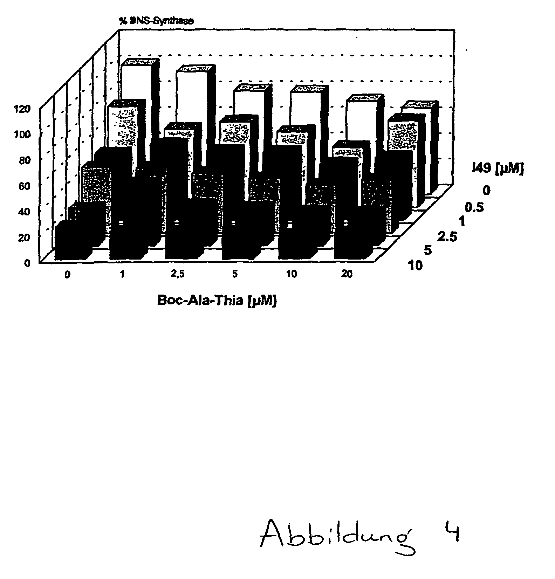

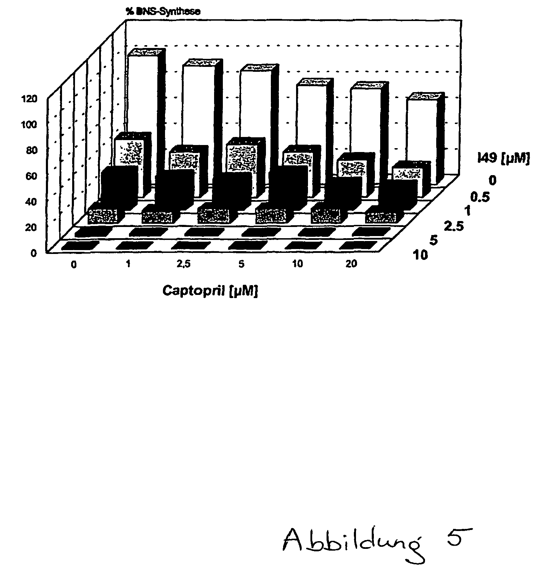

Unsere Untersuchungen zeigten, dass die DNS-Synthese humaner peripherer T-Lymphozyten durch die simultane Administration von Inhibitoren der DP IV (Lys[Z(NO₂)]-thiazolidid = I49) und APN (Actinonin) in superadditiver Weise gehemmt wird. Die T-Zellen wurden 72 h in Gegenwart der genannten Inhibitoren inkubiert und anschließend über die Messung der ³[H]-Thymidin-Inkorporation die DNS-Synthese bestimmt, wie bei Reinhold et al. beschrieben (Reinhold D et al.: Inhibitors of dipeptidyl peptidase IV induce secretion of transforming growth factor β1 in PWM-stimulated PBMNC and T cells. Immunology 1997; 91: 354-360). Abbildung 1 zeigt die dosisabhängige, superadditive Hemmung der DNS-Synthese.

**Abb. 1**: Synergistischer und dosisabhängiger Effekt von Inhibitoren der DP IV (I49) und der Aminopeptidase N (Actinonin) auf die DNS-Synthese humaner T-Lymphozyten. Humane periphere T-Zellen wurden über drei Tage mit den angegebenen Konzentrationen der Inhibitoren inkubiert. Anschließend wurde dem Kulturmedium ³[H]-Methyl-Thymidin zugesetzt und nach weiteren 6 Stunden die in die DNS eingebaute Menge an ³[H]-Thymidin gemessen.

### Beispiel 2

### Inhibierung der DNS-Synthese von humanen peripheren mononukleären Zellen durch Inkubation mit synthetischen Inhibitoren der DP IV und der APN

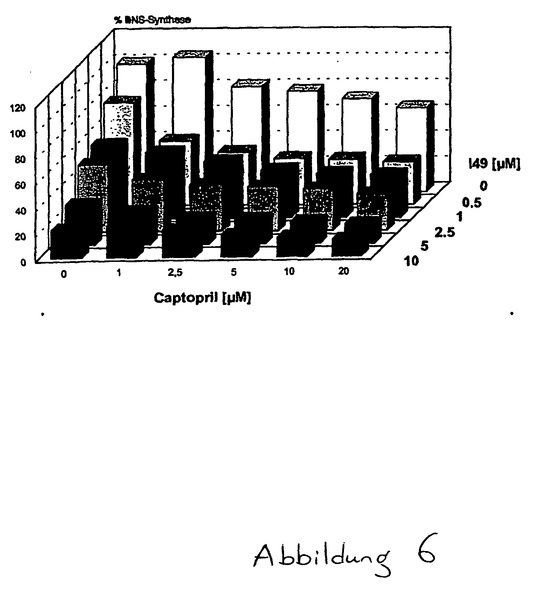

Unsere Untersuchungen zeigten, dass die DNS-Synthese humaner peripherer mononukleärer Zellen (MNZ) durch die simultane Administration von Inhibitoren der DP IV (Lys[Z(NO₂)]-thiazolidid = I49) und APN (Actinonin) in superadditiver Weise gehemmt wird. Die MNZ wurden 72 h in Gegenwart der genannten Inhibitoren inkubiert und anschließend über die Messung der ³[H]-Thymidin-Inkorporation die DNS-Synthese bestimmt, wie bei Reinhold et al. beschrieben (Reinhold D et al.: Inhibitors of dipeptidyl peptidase IV induce secretion of transforming growth factor β1 in PWM-stimulated PBMNC and T cells. Immunology 1997; 91: 354-360). Abbildung 2 zeigt die dosisabhängige, superadditive Hemmung der DNS-Synthese.

**Abb. 2**: Synergistischer und dosisabhängiger Effekt von Inhibitoren der DP IV (I49) und der APN (Actinonin) auf die DNS-Synthese humaner mononukleärer Zellen (MNZ). Humane MNZ wurden über drei Tage mit den angegebenen Konzentrationen der Inhibitoren inkubiert. Anschließend wurde dem Kulturmedium ³[H]-Methyl-Thymidin zugesetzt und nach weiteren 6 Stunden die in die DNS eingebaute Menge an ³[H]-Thymidin gemessen.

### Beispiel 3

### Inhibierung der DNS-Synthese von humanen T-Lymphozyten durch Inkubation mit synthetischen Inhibitoren der DP IV und der POP

Unsere Untersuchungen zeigten, dass die DNS-Synthese humaner T-Lymphozyten durch die simultane Administration von Inhibitoren der DP IV (Lys[Z(NO₂)]-thiazolidid = I49) und der Prolyloligopeptidase (Boc-Ala-Thiazolidid) in superadditiver Weise gehemmt wird. Die T-Zellen wurden 72 h in Gegenwart der genannten Inhibitoren inkubiert und anschließend über die Messung der ³[H]-Thymidin-Inkorporation die DNS-Synthese bestimmt, wie bei Reinhold et al. beschrieben (Reinhold D et al.: Inhibitors of dipeptidyl peptidase IV induce secretion of transforming growth factor β1 in PWM-stimulated PBMNC and T cells. Immunology 1997; 91: 354-360). Abbildung 3 zeigt die dosisabhängige, superadditive Hemmung der DNS-Synthese.

**Abb. 3**: Synergistischer und dosisabhängiger Effekt von Inhibitoren der DP IV (I49) und der Prolyloligopeptidase (Boc-Ala-Thia) auf die DNS-Synthese humaner peripherer T-Lymphozyten. Humane T-Zellen wurden über drei Tage mit den angegebenen Konzentrationen der Inhibitoren inkubiert. Anschließend wurde dem Kulturmedium ³[H]-Methyl-Thymidin zugesetzt und nach weiteren 6 Stunden die in die DNS eingebaute Menge an ³[H]-Thymidin gemessen.

### Beispiel 4

### Inhibierung der DNS-Synthese von humanen peripheren mononukleären Zellen durch Inkubation mit synthetischen Inhibitoren der DP IV und der POP

Unsere Untersuchungen zeigten, dass die DNS-Synthese humaner peripherer mononukleärer Zellen (MNZ) durch die simultane Administration von Inhibitoren der DP IV (Lys[Z(NO₂)]-thiazolidid = I49) und der Prolyloligopeptidase (Boc-Ala-Thiazolidid) in superadditiver Weise gehemmt wird. Die MNZ wurden 72 h in Gegenwart der genannten Inhibitoren inkubiert und anschließend über die Messung der ³[H]-Thymidin-Inkorporation die DNS-Synthese bestimmt, wie bei Reinhold et al. beschrieben (Reinhold D et al.: Inhibitors of dipeptidyl peptidase IV induce secretion of transforming growth factor β1 in PWM-stimulated PBMNC and T cells. Immunology 1997; 91: 354-360). Abbildung 4 zeigt die dosisabhängige, superadditive Hemmung der DNS-Synthese.

**Abb. 4**: Synergistischer und dosisabhängiger Effekt von Inhibitoren der DP IV (I49) und der Prolyloligopeptidase (Boc-Ala-Thia) auf die DNS-Synthese humaner mononukleärer Zellen (MNZ). Humane MNZ wurden über drei Tage mit den angegebenen Konzentrationen der Inhibitoren inkubiert. Anschließend wurde dem Kulturmedium ³[H]-Methyl-Thymidin zugesetzt und nach weiteren 6 Stunden die in die DNS eingebaute Menge an ³[H]-Thymidin gemessen.

### Beispiel 5

### Inhibierung der DNS-Synthese von humanen T-Lymphozyten durch Inkubation mit synthetischen Inhibitoren der DP IV und des ACE

Unsere Untersuchungen zeigten, dass die DNS-Synthese humaner T-Lymphozyten durch die simultane Administration von Inhibitoren der DP IV (Lys[Z(NO₂)]-thiazolidid = I49) und des Angiotensin-konvertierenden Enzyms (Captopril) in superadditiver Weise gehemmt wird. Die T-Zellen wurden 72 h in Gegenwart der genannten Inhibitoren inkubiert und anschließend über die Messung der ³[H]-Thymidin-Inkorporation die DNS-Synthese bestimmt, wie bei Reinhold et al. beschrieben (Reinhold D et al.: Inhibitors of dipeptidyl peptidase IV induce secretion of transforming growth factor β1 in PWM-stimulated PBMNC and T cells. Immunology 1997; 91: 354-360). Abbildung 5 zeigt die dosisabhängige, superadditive Hemmung der DNS-Synthese.

**Abb. 5**: Synergistischer und dosisabhängiger Effekt von Inhibitoren der DP IV (I49) und des Angiotensin-konvertierenden Enzyms (Captopril) auf die DNS-Synthese humaner peripherer T-Lymphozyten. Humane T-Zellen wurden über drei Tage mit den angegebenen Konzentrationen der Inhibitoren inkubiert. Anschließend wurde dem Kulturmedium ³[H]-Methyl-Thymidin zugesetzt und nach weiteren 6 Stunden die in die DNS eingebaute Menge an ³[H]-Thymidin gemessen.

### Beispiel 6

### Inhibierung der DNS-Synthese von humanen peripheren mononukleären Zellen durch Inkubation mit synthetischen Inhibitoren der DP IV und des ACE

Unsere Untersuchungen zeigten, dass die DNS-Synthese humaner peripherer mononukleärer Zellen (MNZ) durch die simultane Administration von Inhibitoren der DP IV (Lys[Z(NO₂)]-thiazolidid = I49) und des Angiotensin-konvertierenden Enzyms (Captopril) in superadditiver Weise gehemmt wird. Die MNZ wurden 72 h in Gegenwart der genannten Inhibitoren inkubiert und anschließend über die Messung der ³[H]-Thymidin-Inkorporation die DNS-Synthese bestimmt, wie bei Reinhold et al. beschrieben (Reinhold D et al.: Inhibitors of dipeptidyl peptidase IV induce secretion of transforming growth factor β1 in PWM-stimulated PBMNC and T cells. Immunology 1997; 91: 354-360). Abbildung 6 zeigt die dosisabhängige, superadditive Hemmung der DNS-Synthese.

**Abb. 6**: Synergistischer und dosisabhängiger Effekt von Inhibitoren der DP IV (I49) und des Aangiotensin-konvertierenden Enzyms (Captopril) auf die DNS-Synthese humaner mononukleärer Zellen (MNZ). Humane MNZ wurden über drei Tage mit den angegebenen Konzentrationen der Inhibitoren inkubiert. Anschließend wurde dem Kulturmedium ³[H]-Methyl-Thymidin zugesetzt und nach weiteren 6 Stunden die in die DNS eingebaute Menge an ³[H]-Thymidin gemessen.

### Beispiel 7

### Hemmung der Proliferation von humanen peripheren mononukleären Zellen (MNZ) durch die einzelne und gleichzeitige Gabe von Hemmstoffen der DP IV (I49 = Lys[Z(NO₂)]-thiazolidid) und APN (Actinonin).

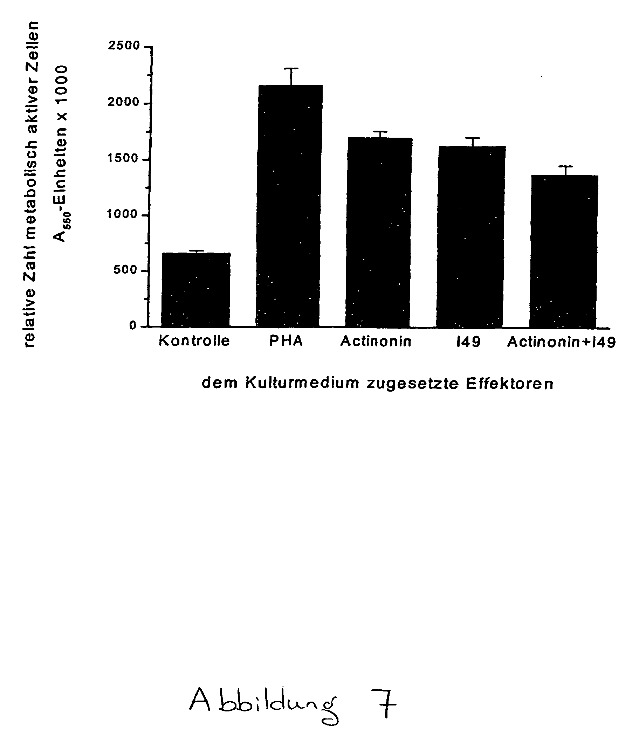

**Abb. 7**: Die MNZ wurden über einen Zeitraum von 72 h ohne Zusatz (Kontrolle), mit dem mitogenen Lektin Phytohämagglutinin (PHA) bzw. mit PHA und den angegebenen Inhibitoren inkubiert. Anschließend erfolgte die Bestimmung der Zahl metabolisch aktiver Zellen unter Verwendung des kommerziell verfügbaren WST-1 Zell-Proliferations-Assays (Takara Inc.) nach Angaben des Herstellers.

### Beispiel 8

### Hemmung der Proliferation der humanen T-Zelllinie KARPAS-299 durch die einzelne und gleichzeitige Gabe von Hemmstoffen der DP IV (I49 = Lys[Z(NO₂)]-thiazolidid) und APN (Actinonin und Probestin).

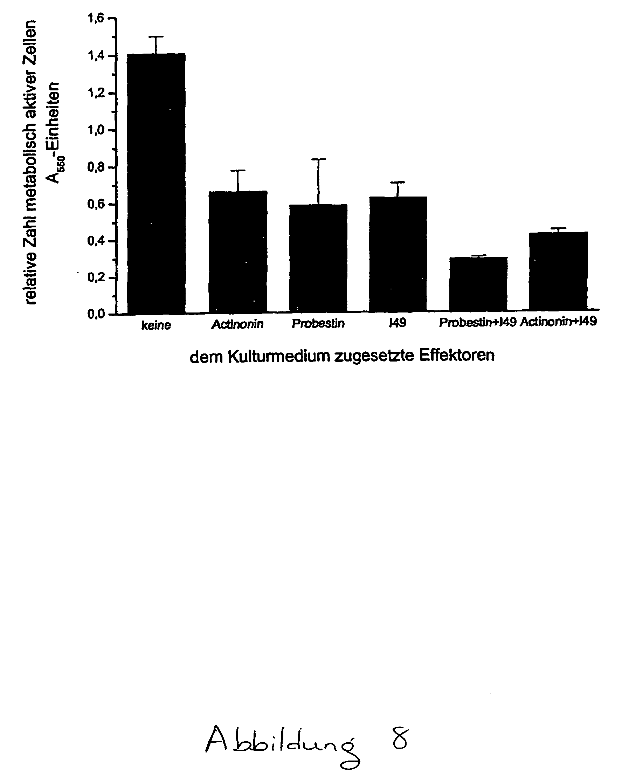

**Abb. 8:** Die KARPAS-299-Zellen wurden über einen Zeitraum von 72 h ohne Zusatz (Kontrolle) bzw. in Gegenwart der angegebenen Inhibitoren einzeln sowie in Kombination inkubiert. Anschließend erfolgte die Bestimmung der Zahl metabolisch aktiver Zellen unter Verwendung des kommerziell verfügbaren WST-1 Zell-Proliferations-Assays (Takara Inc.) nach Angaben des Herstellers.

### Beispiel 9

### Hemmung der Proliferation aktivierter, humaner peripherer T-Zellen durch die einzelne bzw. gleichzeitige Gabe von Hemmstoffen der DP IV (I49 = Lys[Z(NO₂)]-thiazolidid) und APN (Actinonin und Probestin).

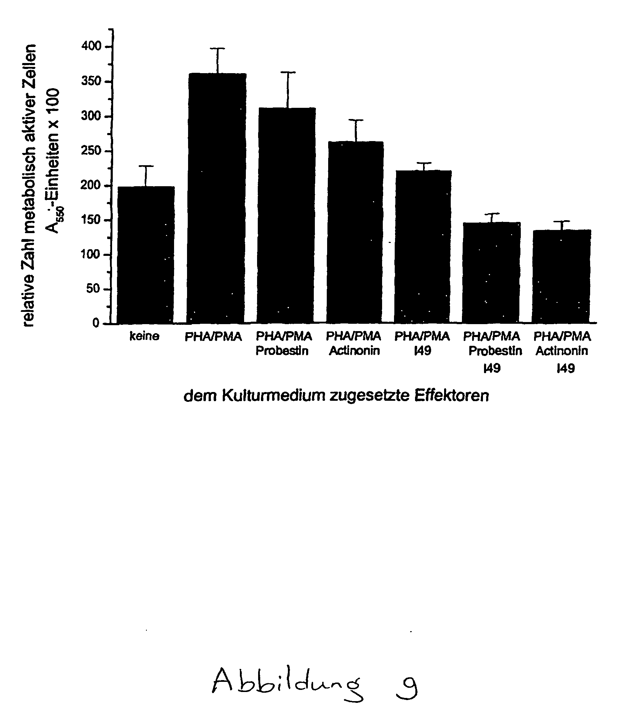

**Abb. 9**: Die T-Zellen wurden mit Ausnahme der unbehandelten Kontrolle durch Zugabe zum Kulturmedium von Phytohämagglutinin und Phorbol-12-myristat-13-acetat aktiviert und über einen Zeitraum von 72 h in Gegenwart der angegebenen Inhibitoren einzeln sowie in Kombination inkubiert. Anschließend erfolgte die Bestimmung der Zahl metabolisch aktiver Zellen unter Verwendung des kommerziell verfügbaren WST-1 Zell-Proliferations-Assays (Takara Inc.) nach Angaben des Herstellers.

### Beispiel 10

### Hemmung der Proliferation PHA-aktivierter, humaner mononukleärer Zellen (MNZ) durch die einzelne bzw. gleichzeitige Gabe von Hemmstoffen der DP IV (I49 = Lys[Z(NO₂)]-thiazolidid) und der X-Pro-Aminopeptidase (APP) (Apstatin).

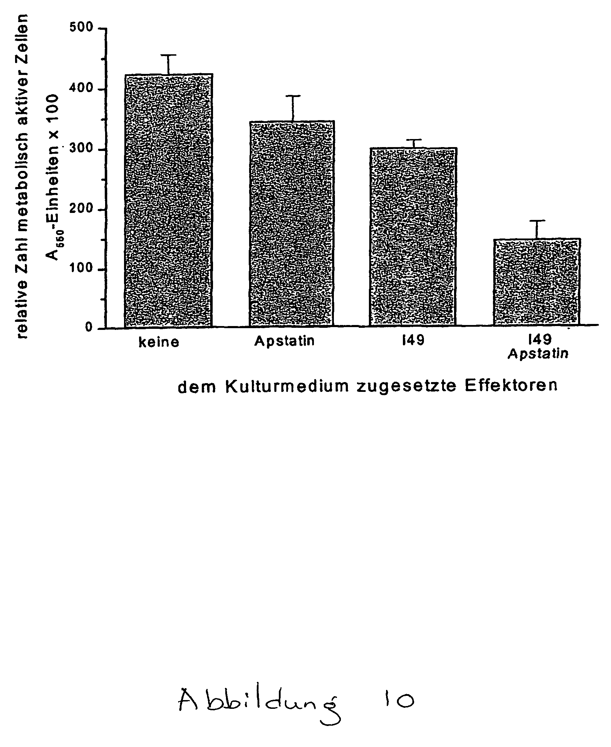

**Abb. 10**: Die mononukleären Zellen (MNZ) wurden über einen Zeitraum von 72 h in Gegenwart der angegebenen Inhibitoren einzeln sowie in Kombination inkubiert. Anschließend erfolgte die Bestimmung der Zahl metabolisch aktiver Zellen unter Verwendung des kommerziell verfügbaren WST-1 Zell-Proliferations-Assays (Takara Inc.) nach Angaben des Herstellers.

## Patentansprüche

1. Verwendung von Inhibitoren der Dipeptidylpeptidase IV (DP IV) sowie von Inhibitoren von Enzymen mit gleicher Substratspezifität (DP IV-analoge Enzymaktivität) in Kombination mit Inhibitoren der Alanyl-Aminopeptidase (Aminopeptidase N, APN) bzw. mit Inhibitoren von Enzymen gleicher Substratspezifität (APN-analoge Enzymaktivität), Inhibitoren der X-Pro-Aminopeptidase (Aminopeptidase P, APP), Inhibitoren des "angiotensin-converting enzyme" (ACE) und/oder Inhibitoren der Prolyloligopeptidase (POP, Prolylendopeptidase, PEP) zur Herstellung eines Arzneimittels zur Vorbeugung und Therapie von Autoimmunerkrankungen und/oder zur Unterdrückung von Transplantat-Abstossung und/oder zur Therapie von Tumorerkrankungen.

2. Verwendung nach Anspruch 1, worin die Inhibitoren der DP IV Xaa-Pro-Dipeptide (Xaa = α-Aminosäure bzw. seitenkettengeschütztes Derivat), entsprechende Derivate, vorzugsweise Dipeptidphosphonsäurediarylester, Dipeptidboronsäuren (z.B. Pro-boro-Pro) und deren Salze, Xaa-Xaa-(Trp)-Pro-(Xaa)n-Peptide (Xaa = α-Aminosäure, n=0-10), entsprechende Derivate und deren Salze bzw. Aminosäure (Xaa)-amide, entsprechende Derivate und deren Salze sind, wobei Xaa eine α-Aminosäure bzw. ein seitenkettengeschütztes Derivat, vorzugsweise N^{ε}-4-Nitrobenzyloxycarbonyl-L-Lysin, L-Prolin, L-Tryptophan, L-Isoleucin, L-Valin ist und als Amidstruktur cyclische Amine, z.B. Pyrrolidin, Piperidin, Thiazolidin und deren Derivate fungieren.

3. Verwendung nach Anspruch 1 oder Anspruch 2, worin Aminosäureamide, z.B. N^{ε}-4-Nitrobenzyloxy-carbonyl-L-Lysin-thiazolidid, -pyrrolidid und -piperidid sowie das entsprechende 2-Cyanothiazolidid-, 2-Cyanopyrrolidid- und 2-Cyanopiperididderivat als DP IV-Inhibitoren eingesetzt werden.

4. Verwendung nach einem der Ansprüche 1 bis 3, wobei als Inhibitoren der APN Actinonin, Leuhistin, Phebestin, Amastin, Probestin β-Aminothiole, α-Aminophosphinsäuren, α-Amino-phosphinsäurederivate, vorzugsweise D-Phe-ψ[PO(OH)-CH₂]-Phe-Phe und deren Salze,
als Inhibitoren der APP Apstatin, (2S,3R)-HAMH-L-Prolin, (2S,3R)-HAPB-L-Prolin, die entsprechenden L-Prolinmethylester, (2S,3R)-HANM-/(2S,3R)-HAPB-pyrrolidide, -thiazolidide (HAMH = 3 Amino-2hydroxy-5-methyl-hexanoyl, HAPB = 3-Amino-2-hydroxy-4-phenyl-butanoyl) und der Salze,
als Inhibitoren des ACE Captopril, Enalapril, Lisinopril, Cilazopril und deren Salze,
als Inhibitoren der POP (PEP) Postatin, Eurystatin A oder B, N^{a}-geschützte Peptidaldehyde, vorzugsweise Benzyloxycarbonyl-L-Prolyl-L-Prolinal bzw. Benzyloxycarbonyl-L-Thioprolyl-L-Thioprolinal, N^{a}-geschützte Aminosäure(Xaa)-pyrrolidide bzw. -thiazolidide (Xaa = α-Aminosäure, bevorzugt L-Alanin, L-Valin, L-Isoleucin) sowie die entsprechenden 2-Cyanopyrrolidid- bzw. 2-Cyanothiazolididderivate, substratanaloge N^{a}-geschützte Peptidphosphonsäurediarylester bzw. Peptiddiazomethylketone bzw. Peptidammoniummethylketone und deren Salze fungieren.

5. Verwendung von Inhibitorkombinationen nach einem der Ansprüche 1 bis 4 zur Vorbeugung und Therapie von Rheumatoider Arthritis, Lupus erythematodes, Multipler Sklerose, IDDM, Morbus Crohn, Colitis Ulcerosa, Psoriasis, Neurodermitis, Glumerulonephritis, interstitielle Nephritis, Vaskulitis, autoimmunen Schilddrüsenerkrankungen oder autoimmunhämolytischer Anämie, sowie anderen chronischen Erkrankungen mit entzündlicher Genese wie Allergien und Arteriosklerose.

6. Pharmazeutische Zubereitungen, umfassend Inhibitoren der Dipeptidylpeptidase IV (DP IV) oder Inhibitoren von Enzymen mit DP IV-analoger Enzymaktivität in Kombination mit Inhibitoren eines der Enzyme Alanyl-Aminopeptidase (Aminopeptidase N, APN) bzw. Enzyme gleicher Substratspezifität (APN-analoge Enzymaktivität), X-Pro-Aminopeptidase (Aminopeptidase P, APP), "angiotensin-converting enzyme" (ACE) und/oder Prolyloligopeptidase (POP, Prolylendopeptidase, PEP) und in Kombination mit an sich bekannten Träger-, Zusatz- und/oder Hilfsstoffen.

7. Pharmazeutische Zubereitung nach Anspruch 6, umfassend als Inhibitoren der DP IV Xaa-Pro-Dipeptide (Xaa = α-Aminosäure bzw. seitenkettengeschützte Derivate), entsprechende Derivate, vorzugsweise Dipeptidphosphonsäurediarylester, Dipeptidboronsäuren (z. B. Pro-boro-Pro) und deren Salze, Xaa-Xaa-(Trp)-Pro-(Xaa)n-Peptide (Xaa = α-Aminosäuren, n=0-10), entsprechende Derivate und deren Salze bzw. Aminosäure (Xaa)-amide, entsprechende Derivate und deren Salze, wobei Xaa eine α-Aminosäure bzw. deren seitenkettengeschütztes Derivat, vorzugsweise N^{ε}-4-Nitrobenzyloxycarbonyl-L-Lysin, L-Prolin, L-Tryptophan, L-Isoleucin, L-Valin, ist und als Amidstruktur cyclische Amine, z.B. Pyrrolidin, Piperidin, Thiazolidin und deren Derivate fungieren.

8. Pharmazeutische Zuberetung nach Anspruch 7, umfassend als Inhibitoren der DP IV Aminoasäureamide, z.B. N^{ε}-4-Nitrobenzyloxy-carbonyl-L-Lysin-thiazolidid, -pyrrolidid und -piperidid sowie das entsprechende 2-Cyanothiazolidid-, 2-Cyanopyrrolidid- und 2-Cyanopiperididderivat.

9. Pharmazeutische Zubereitung nach Anspruch 7, umfassend die Inhibitoren Actinonin, Leuhistin, Phebestin, Amastin, Probestin, β-Aminothiole, α-Aminophosphinsäuren, α-Aminophosphinsäurederivate bevorzugt D-Phe-ψ[PO(OH)-CH₂]-Phe-Phe und deren Salze als APN-Inhibitoren, Apstatin, (2S,3R)-HANM-L-Prolin, (2S,3R)-HAPB-L-Prolin, die entsprechenden L-Prolinmethylester, (2S,3R)-HAMH/(2S,3R)-HAPB-pyrrolidide, -thiazolidide (HAMH = 3-Amino-2-hydroxy-5-methyl-hexanoyl, HAPB = 3-Amino-2-hydroxy-4-phenyl-butanoyl) und der Salze als APP-Inhibitoren, Captopril, Enalapril, Lisinopril, Cilazopril und deren Salze als ACE-Inhibitoren, Postatin, Eurystatin A oder B, N^{a}-geschützte Peptidaldehyde, vorzugsweise Benzyloxycarbonyl-L-Prolyl-L-Prolinal bzw. Benzyloxycarbonyl-L-Thioprolyl-L-Thioprolinal, N^{a}-geschützte Aminosäure(Xaa)-pyrrolidide bzw. -thiazolidide (Xaa = α-Aminosäure, bevorzugt L-Alanin, L-Valin, L-Isoleucin) sowie die entsprechenden 2-Cyanopyrrolidid- bzw. 2-Cyanothiazolididderivate, substratanaloge N^{a}-geschützte Peptidphosphonsäurediarylester bzw. Peptiddiazomethylketone bzw. Peptidammoniummethylketone und deren Salze als POP (PEP)-Inhibitoren.

10. Pharmazeutische Zubereitung nach einem der Ansprüche 6 bis 9, umfassend zwei oder mehrere der Inhibitoren der DP IV bzw. DP IV-analoger Enzymaktivität, der APN bzw. APN-analoger Enzymaktivität, des ACE, der POP (PEP) und der APP in räumlich getrennter Formulierung in Kombination mit an sich bekannten Träger-, Hilfs- und/oder Zusatzstoffen zur gleichzeitigen oder zeitlich unmittelbar aufeinanderfolgenden Verabreichung mit dem Ziel einer gemeinsamen Wirkung.

11. Pharmazeutische Zubereitung nach einem der Ansprüche 6 bis 10 für die systemische Anwendung zur oralen, transdermalen, intravenösen, subcutanen, intracutanen, intramuskulären, rektalen, vaginalen, sublingualen Applikation zusammen mit an sich bekannten Träger-, Hilfs- und/oder Zusatzstoffen.

12. Pharmazeutische Zubereitung nach einem der Ansprüche 6 bis 10 für die topische Anwendung in Form von z.B. Cremes, Salben, Pasten, Gelen, Lösungen, Sprays, Liposomen, Schüttelmixturen, Hydrokolloidverbänden bzw. andern dermatologischen Grundlagen/Vehikeln, einschließlich instillativer Applikation.

## Claims

1. Use of inhibitors of dipeptidyl peptidase IV (DP IV) as well as of inhibitors of enzymes having the same substrate specificity (DP IV-analogous enzyme activity) in combination with inhibitors of alanyl aminopeptidase (aminopeptidase N, APN) as well as with inhibitors of enzymes having the same substrate specificity (APN-analogous enzyme activity), with inhibitors of X-Pro-aminopeptidase (aminopeptidase P, APP), with inhibitors of the angiotensin-converting enzyme (ACE) and/or with inhibitors of prolyl oligopeptidase (POP, prolyl endopeptidase, PEP) for the manufacture of a medicament for a prevention and therapy of autoimmune diseases and/or for a suppression of transplant rejection and/or for a therapy of tumor diseases.

2. Use according to claim 1, wherein the inhibitors of DP IV are Xaa-Pro-dipeptides (Xaa = α-amino acid or side chain-protected derivative), corresponding derivatives, preferably dipeptide phosphonic acid diaryl esters, dipeptide boronic acids (e. g. Pro-boro-Pro) and salts thereof, Xaa-Xaa-(Trp)-Pro-(Xaa)ₙ-peptides (Xaa = α-amino acid, n = 0 - 10), corresponding derivatives and salts thereof or amino acid (Xaa)-amides, corresponding derivatives and salts thereof, wherein Xaa is an α-amino acid or a side chain-protected derivative, respectively, preferably N^{∈}-4-nitrobenzyloxycarbonyl L-lysine, L-proline, L-tryptophane, L-isoleucine, L-valine, and cyclic amines as, for example, pyrrolidine, piperidine, thiazolidine and derivatives thereof act as amide structure.

3. Use according to claim 1 or claim 2, wherein amino acid amides, e. g. N^{∈}-4-nitrobenzyloxycarbonyl-L-lysine-thiazolidid, -pyrrolidid and -piperidid as well as the corresponding 2-cyanothiazolidid, 2-cyanopyrrolidid and 2-cyanopiperidid derivative, are employed as the DP IV inhibitor.

4. Use according to any of the claims 1 to 3, wherein Actinonin, Leuhistin, Phebestin, Amastin, Probestin, β-aminothiols, α-amino phosphinic acids, α-amino phoshinic acid derivatives, preferably D-Phe-ψ-[PO(OH)-CH₂]-Phe-Phe and salts thereof, act as inhibitors of APN;
Apstatin, (2S,3R)-HAMH-L-proline, (2S,3R)-HAPB-L-proline, the corresponding L-proline methyl esters, (2S,3R)-HAMH/(2S,3R)-HAPB-pyrrolidides, -thiazolidides (HAMH = 3-amino-2-hydroxy-5-methyl hexanoyl, HAPB = 3-amino-2-hydroxy-4-phenyl butanoyl) and salts thereof act as inhibitors of APP;
Captopril, Enalapril, Lisinopril, Cilazopril and salts thereof act as inhibitors of ACE;
Postatin, Eurystatin A or B, N^{a}-protected peptide aldehydes, preferably benzyloxycarbonyl-L-prolyl-L-prolinal or benzyloxycarbonyl-L-thioprolyl-L-thioprolinal, N^{a}-protected amino acid (Xaa)-pyrrolidides or -thiazolidides (Xaa = α-amino acid, preferably L-alanine, L-valine, L-isoleucine) as well as the corresponding 2-cyanopyrrolidid or 2-cyanothiazolidid derivatives, substrate-analogous N^{a}-protected peptide phosphonic acid diaryl esters or peptid diazo methyl ketones or peptide ammonium methyl ketones and salts thereof act as inhibitors of POP (PEP).

5. Use of inhibitor combinations of any of claims 1 to 4 for a prevention and therapy of Rheumatoid Arthritis, Lupus Erythematodes, Multiple Sclerosis, IDDM, Morbus Crohn, Colitis Ulcerosa, Psoriasis, Neurodermitis, Glomerulonephritis, Interstitial Nephritis, Vasculitis, autoimmune diseases of the thyroid gland or autoimmune hemolytic anemia as well as of other chronic diseases with an inflammatory genesis as, for example, allergies and arteriosclerosis.

6. Pharmaceutical preparations, comprising inhibitors of dipeptidyl peptidase IV (DP IV) or inhibitors of emzymes having DP IV-analogous enzyme activity in combination with inhibitors of any of the enzymes alanyl aminopeptidase (aminopeptidase N, APN) as well as of enzymes having the same substrate specificity (APN-analogous enzyme activity), of X-Pro-aminopeptidase (aminopeptidase P, APP), of the angiotensin-converting enzyme (ACE) and/or of prolyl oligopeptidase (POP, prolyl endopeptidase, PEP) and in combination with per se known carrier, additive and/or auxiliary substances.

7. Pharmaceutical preparation according to claim 6, comprising Xaa-Pro-dipeptides (Xaa = α-amino acid or side chain-protected derivatives) corresponding derivatives, preferably dipeptide phosphonic acid diaryl esters, dipeptide boronic acids (e. g. Pro-boro-Pro) and salts thereof, Xaa-Xaa-(Trp)-Pro-(Xaa)ₙ-peptides (Xaa = α-amino acid, n = 0 - 10), corresponding derivatives and salts thereof or amino acid (Xaa)-amides, corresponding derivatives and salts thereof, wherein Xaa is an α-amino acid or a side chain-protected derivative, respectively, preferably N^{∈}-4-nitrobenzyloxycarbonyl L-lysine, L-proline, L-tryptophane, L-isoleucine, L-valine, and cyclic amines as, for example, pyrrolidine, piperidine, thiazolidine and derivatives thereof act as amide structure, as the inhibitors of DP IV.

8. Pharmaceutical preparation according to claim 7, comprising amino acid amides, e. g. N^{∈}-4-nitrobenzyloxycarbonyl-L-lysine-thiazolidid, -pyrrolidid and -piperidid as well as the corresponding 2-cyanothiazolidid, 2-cyanopyrrolidid and 2-cyanopiperidid derivative as the inhibitors of DP IV.

9. Pharmaceutical preparation according to claim 7, comprising the inhibitors
Actinonin, Leuhistin, Phebestin, Amastin, Probestin, β-aminothiols, α-amino phosphinic acids, α-amino phoshinic acid derivatives, preferably D-Phe-ψ-[PO(OH)-CH₂]-Phe-Phe and salts thereof, as the inhibitors of APN;
Apstatin, (2S,3R)-HAMH-L-proline, (2S,3R)-HAPB-L-proline, the corresponding L-proline methyl esters, (2S,3R)-HAMH- / (2S,3R)-HAPB-pyrrolidides, -thiazolidides (HAMH = 3-amino-2-hydroxy-5-methyl hexanoyl, HAPB = 3-amino-2-hydroxy-4-phenyl butanoyl) and salts thereof as the inhibitors of APP;
Captopril, Enalapril, Lisinopril, Cilazopril and salts thereof as the inhibitors of ACE;
Postatin, Eurystatin A or B, N^{a}-protected peptide aldehydes, preferably benzyloxycarbonyl-L-prolyl-L-prolinal or benzyloxycarbonyl-L-thioprolyl-L-thioprolinal, N^{a}-protected amino acid (Xaa)-pyrrolidides or -thiazolidides (Xaa = α-amino acid, preferably L-alanine, L-valine, L-isoleucine) as well as the corresponding 2-cyanopyrrolidid or 2-cyanothiazolidid derivatives, substrate-analogous N^{a}-protected peptide phosphonic acid diaryl esters or peptid diazo methyl ketones or peptide ammonium methyl ketones and salts thereof as inhibitors of POP (PEP).

10. Pharmaceutical preparation according to any of the claims 6 to 9, comprising two or more of the inhibitors of DP IV or of enzymes having a DP IV-analogous enzyme activity, of APN or of enzymes having an APN-analogous enzyme activity, of ACE, of POP (PEP) and of APP in a spaced apart formulation in combination with per se known carrier, auxiliary and/or additive substances for a simultaneous or a directly consecutive administration with the aim of a combined action.

11. Pharmaceutical preparation according to claims 6 to 10 for a systemic use for the oral, transdermal, intravenous, subcutaneous, intracutaneous, intramuscular, rectal, vaginal, sublingual application, together with per se known carrier, auxiliary and/or additive substances.

12. Pharmaceutical preparation according to claims 6 to 10 for a topical use in the form of, for example, creams, ointments, pastes, gels, solutions, sprays, liposomes, shaked mixtures, hydrocolloid dressings, and other dermatological bases/vehicles including instillative applications.

## Revendications

1. Utilisation d'inhibiteurs de la dipeptidylpeptidase IV (DP IV), ainsi que d'inhibiteurs d'enzymes ayant la même spécificité de substrat (activité enzymatique analogue à la DP IV) en combinaison avec des inhibiteurs de l'analyl-aminopeptidase (aminopeptidase N, APN) ou avec des inhibiteurs d'enzymes ayant la même spécificité de substrat (activité enzymatique analogue à l'APN), des inhibiteurs de la X-Pro-aminopeptidase (aminopeptidase P, APP), des inhibiteurs de l'enzyme de conversion de l'angiotensine (ECA) et/ou des inhibiteurs de la prolyloligopeptidase (POP), prolylendopeptidase, PEP) pour préparer un médicament destiné à prévenir et traiter des maladies auto-immunes et/ou pour supprimer le rejet de greffons et/ou pour le traitement de maladies tumorales.

2. Utilisation selon la revendication 1, pour laquelle les inhibiteurs de la DP IV sont les Xaa-Pro-dipeptides (Xaa = acides α-aminés ou dérivés protégés en chaîne latérale) des dérivés correspondants, de préférence des esters diaryliques d'acides dipeptidephosphoniques, des acides dipeptideboroniques (par exemple, Pro-boro-Pro) et leurs sels, des Xaa-Xaa-(trp)-Pro-(Xaa)n-peptides (Xaa = acides α-aminés, n = 0 à 10), des dérivés correspondants et leurs sels ou des amides d'acides aminés (Xaa), des dérivés correspondants et leurs sels, où Xaa est un acide α-aminé ou un dérivé protégé en chaîne latérale, de préférence la N^{ε}-4-nitrobenzyloxycarbonyl-L-lysine, la L-proline, le L-tryptophane, la L-isoleucine, la L-valine, les composés jouant le rôle de structure amide étant des amines cycliques, par exemple, la pyrrolidine, la pipéridine, la thiazolidine et leurs dérivés.

3. Utilisation selon la revendication 1 ou 2, pour laquelle on utilise des amides d'acides aminés, par exemple, le thiazolidide, le pyrrolidide et le pipéridide de la N^{ε}-4-nitrobenzyloxycarbonyl-L-lysine, ainsi que le dérivé correspondant 2-cyanothiazolidide, 2-cyanopyrrolidide et 2-cyanopipéridide, en tant qu'inhibiteurs de la DP IV.

4. Utilisation selon l'une des revendications 1 à 3, pour laquelle on utilise tant qu'inhibiteurs de l'APN l'actinonine, la leuhistine, la phebestine, l'amastine, la probestine, les β-aminothiols, les acides α-aminophosphiniques, les dérivés d'acides α-aminophosphiniques, de préférence le D-Phe-ψ[PO(OH)-CH₂]-Phe-Phe et leurs sels,
en tant qu'inhibiteurs de l'APP, l'apstatine, la (2S, 3R)-HAMH-L-proline, la (2S, 3R)-HAPB-L-proline, les esters méthyliques correspondants de la L-proline, le pyrrolidide et le thiazolidide de (2S, 3R)-HAMH-/(2S, 3R)-HAPB (HAMH = 3-amino-2-hydroxy-5-méthyl-hexanoyle, HAPB = 3-amino-2-hydroxy-4-phényl-butanoyle) et les sels,
en tant qu'inhibiteurs de l'ECA, le captopril, l'énalapril, le lisinopril, le cilazopril et leurs sels,
en tant qu'inhibiteurs de la POP (PEP) la postatine, l'eurystatine A ou B, les peptide-aldéhydes protégés en N^{a}, de préférence le benzyloxycarbonyl-L-prolyl-L-prolinal ou le benzyloxycarbonyl-L-thiopropyl-L-thioprolinal, les pyrrolidides et thiazolidides d'acides aminés (Xaa) protégés en N^{a} (Xaa = acides α-aminés, de préférence la L-alanine, la L-valine, la L-isoleucine), ainsi que les dérivés correspondants 2-cyanopyrrolidides et 2-cyanothiazolidides, les esters diaryliques d'acides peptide-phosphoniques protégés en N^{a} analogues de substrat, ou les peptide-diazométhylcétones ou peptide-ammonium-méthylcétones et leurs sels.

5. Utilisation de combinaisons d'inhibiteurs selon l'une des revendications 1 à 4 pour prévenir et traiter la polyarthrite rhumatoïde, le lupus érythémateux disséminé, la sclérose en plaques, le DID, la maladie de Crohn, la colite ulcéreuse, le psoriasis, la neurodermite, la glomérulonéphrite, la néphrite interstitielle, la vasculite, les maladies auto-immunes de la glande thyroïde ou l'anémie auto-immuno hémolytique, ainsi que d'autres maladies chroniques présentant une genèse inflammatoire, telles que les allergies et l'artériosclérose.

6. Préparation pharmaceutique comprenant des inhibiteurs de la dipeptidylpeptidase IV (DP IV) ou des inhibiteurs d'enzymes ayant une activité enzymatique analogue à la DP IV en combinaison avec des inhibiteurs de l'une des enzymes alanyl-aminopeptidase (aminopeptidase N, APN) ou de l'une des enzymes ayant une spécificité de substrat identique (activité enzymatique analogue à l'APN), la X-Pro-aminopeptidase (aminopeptidase P, APP), l'enzyme de conversion de l'angiotensine (ECA) et/ou la prolyloligopeptidase (POP, prolylendopeptidase, PEP), et en combinaison avec des excipients, additifs et/ou adjuvants connus en soi.

7. Préparation pharmaceutique selon la revendication 6, comprenant en tant qu'inhibiteurs de la DP IV Xaa-Pro-dipeptides, (Xaa = acides α-aminés ou dérivés protégés en chaîne latérales), les dérivés correspondants, de préférence les esters diaryliques des acides dipeptidephosphoniques, les acides dipeptideboroniques, (par exemple, le Pro-boro-Pro) et leurs sels, les Xaa-Xaa-(trp)-Pro-(Xaa)n-peptides (Xaa = acides α-aminés, n = 0 à 10), les dérivés correspondants et leurs sels, ou encore les amides d'acides aminés (Xaa), les dérivés correspondants et leurs sels, où Xaa est un acide α-aminé ou un dérivé protégé en chaîne latérale de ce dernier, de préférence la N^{ε}-4-nitrobenzyloxycarbonyl-L-lysine, la L-proline, le L-tryptophane, la L-isoleucine, la L-valine, les composés à structure amide étant des amines cycliques, par exemple, la pyrrolidine, la pipéridine, la thiazolidine et leurs dérivés.

8. Préparation pharmaceutique selon la revendication 7, comprenant en tant qu'inhibiteurs de la DP IV des amides d'acides aminés, par exemple le thizolidide, le pyrrolidide et le pipéridide de la N^{ε}-4-nitrobenzyloxycarbonyl-L-lysine, ainsi que le dérivé correspondant 2-cyanothiazolidide, 2-cyanopyrrolidide et 2-cyanopipéridide.

9. Préparation pharmaceutique selon la revendication 7, qui comprend les inhibiteurs actinonine, leuhistine, phebestine, amastine, probestine, les β-aminothiols, les acides α-aminophosphiniques, les dérivés des acides α-aminophosphiniques, de préférence le D-Phe-ψ[PO(OH)-CH₂]-Phe-Phe et leurs sels en tant qu'inhibiteurs de l'APN ; l'apstatine, la (2S, 3R)-HAMH-L-proline, la (2S, 3R)-HAPB-L-proline, les esters méthyliques correspondants de la L-proline, les pyrrolidides et thiazolidides du (2S, 3R)-HAMH-/(2S, 3R)-HAPB (HAMH = 3-amino-2-hydroxy-5-méthyl-hexanoyle, HAPB = 3-amino-2-hydroxy-4-phényl-butanoyle) et les sels en tant qu'inhibiteurs de l'APP ; le captopril, l'énalapril, le lisinopril, le cilazopril et leurs sels en tant qu'inhibiteurs de l'ECA ; la postatine, l'eurystatine A ou B, les peptide-aldéhydes protégés en N^{a}, de préférence le benzyloxycarbonyl-L-prolyl-L-prolinal ou le benzyloxycarbonyl-L-thiopropyl-L-thioprolinal, les pyrrolidides et thiazolidides d'acides aminés (Xaa) protégés en N^{a} (Xaa = acides α-aminés, de préférence la L-alanine, la L-valine, la L-isoleucine), ainsi que les dérivés correspondants 2-cyanopyrrolidides et 2-cyanothiazolidides, les esters diaryliques d'acides peptidephosphoniques protégés en N^{a} analogues de substrat, ou les peptide-diazométhylcétones ou les peptide-ammonium-méthylcétones et leurs sels en tant qu'inhibiteurs de la POP (PEP).

10. Préparation pharmaceutique selon l'une des revendications 6 à 9, comprenant deux ou plusieurs des inhibiteurs de la DP IV ou de l'activité enzymatique analogue à la DP IV, de l'APN ou de l'activité enzymatique analogue à l'APN, de l'ECA, du POP (PEP) et de l'APP dans le cadre d'une formulation spatialement distincte en combinaison avec des excipients, adjuvants et/ou additifs connus en soi, pour administration simultanée, ou immédiatement successive, l'objectif étant un effet commun.

11. Préparation pharmaceutique selon l'une des revendications 6 à 10 pour une utilisation systémique pour administration orale, transdermique, intraveineuse, sous-cutanée, intra-cutanée, intramusculaire, rectale, vaginale, sublinguale, en même temps que des excipients, adjuvants et/ou additifs connus en soi.

12. Préparation pharmaceutique selon l'une des revendications 6 à 10 pour utilisation topique sous forme par exemple de crèmes, de pommades, de pâtes, de gels, de solutions, de sprays, de liposomes, de mixtures à agiter avant l'emploi, de pansements aux hydrocolloïdes, ou d'autres bases/véhicules dermatologiques, y compris une administration par instillation.
